# EUROPEAN PATENT APPLICATION

(11) **EP 4 744 577 A1**
(43) Date of publication of application: **20.05.2026**
(21) Application number: 24212698.5
(22) Date of filing: 13.11.2024
(51) Int. Cl.: A61B 5/00, G01N 21/00, G01J 1/00, G01J 3/10, A61B 5/1455, G01J 3/02, G01N 21/47

(54) **MEASUREMENT SYSTEM FOR SKIN-LAYER SPECIFIC NIR SPECTROSCOPY**

(71) Applicant: trinamiX GmbH, 67063 Ludwigshafen am Rhein (DE)
(72) Inventor: SCHMIDT, Felix, 67059 Ludwigshafen am Rhein (DE); OEGUEN, Celal Mohan, 67059 Ludwigshafen am Rhein (DE); LOEFFLER, Jan Gerrit, 60431 Frankfurt (DE); SEIDEL, Sebastian, 67059 Ludwigshafen am Rhein (DE); LEHNERT, Tibor Peter, 67059 Ludwigshafen am Rhein (DE); SHEERAN, Bridget, 67059 Ludwigshafen am Rhein (DE); UNRUH, Tobias, 35781 Weilburg (DE); BAUMGARTNER, Tobias, 67059 Ludwigshafen am Rhein (DE)
(74) Representative: Altmann Stößel Dick Patentanwälte PartG mbB

(57) **Abstract**

A spectrometer (110) and a method for skin-layer specific spectroscopy are disclosed. The spectrometer (110) comprises
- at least one illumination source (112) configured for illuminating a sample (114) of a subject with at least one illumination light (116) beam having at least one wavelength in an optical spectral range, wherein the illumination source (112) is configured for transmitting the illumination light beam (116) to the sample (114) along a direction of propagation (120);
- at least one detector (124) sensitive to light in the optical spectral range, wherein the detector (124) is configured for detecting light impinging on the detector (124) under at least one direction of collection (126);
wherein the illumination source (112) and the detector (124) are separated by a baseline (130), wherein the direction of collection (126) and the direction of propagation (120) of the illumination light beam (116) form an angle of inclination α with α ≠ 0.

## Description

### Technical Field

The invention relates to a spectrometer and a method for skin-layer specific spectroscopy. The invention further relates to a computer program, a computer-readable storage medium and to a non-transient computer-readable storage medium for performing the method. Such devices and methods can, in general, be used for investigating or monitoring purposes, in particular, in the infrared (IR) spectral region, especially in the near-infrared (NIR) spectral region, e.g. for investigating or monitoring of spectroscopic properties of one or more skin layers. However, further application are also feasible.

### Background art

Spectrometers are known to be efficient tools for obtaining information on the spectral properties of an object, when emitting, irradiating, reflecting and/or absorbing light. Spectrometers, thus, may assist in analyzing samples or other tasks in which information on the spectral properties of an object is of interest.

For example, near infrared (NIR) spectroscopy in a wavelength range of 750 to 2500 nm is a known technique used for analyzing molecular vibrations and rotations, as well as combinations and overtones of the molecular vibrations and rotations. NIR spectroscopy may allow for a nondestructive analysis of solid, liquid, and gaseous samples, and may be used in various fields of technology, such as pharmaceuticals, food and agriculture, material science, biomedical research and the like. In general, NIR spectroscopy may be used to analyze the composition of the skin, including lipids, collagen, and elastin level. Alternatively, NIR spectroscopy is known to measure biomarkers in various biological samples, such as skin, blood, urine, and saliva. These techniques can e.g. be used to quantify biomarkers related to disease diagnosis, monitoring of treatment efficacy, and assessment of physiological processes in the body or the like.

Despite the advantages achieved by known methods and devices, several technical challenges remain. The use of spectroscopic techniques for analyzing biomarkers in the skin of a human or animal body may still be technically challenging. Specifically, the NIR spectrum of skin may show many dependencies and variabilities, such as inter-subject and intra-subject variabilities. The isolation of spectral features of low-concentration skin biomarkers over a broadly varying spectral background using proper optical combination of sensors, optical setup or chemometrics tools may therefore pose a technical challenge. In particular, assessing the skin structure via spectroscopic techniques may be associated with several technical challenges. The skin is a multilayered tissue with various components, such as lipids, proteins, and water, which may affect the accuracy of spectroscopic biomarker measurement. Specifically, the skin can be modeled by an epidermis layer, a dermis layer and a subcutaneous layer. For spectroscopic biomarker measurement, signals from the subcutaneous layer may be envisaged since blood vessels are present in this skin layer. In particular, an effective sample of blood vessels in the subcutaneous layer using spectroscopic measurements, e.g. near infrared spectroscopy, is technically still challenging due to the limited penetration depth of light, e.g. of near infrared light of a wavelength of 1450 nm being only a few hundred micrometers in the skin.

### Problem to be solved

It is therefore desirable to provide devices and method which at least partially address above-identified technical challenges. Specifically, a spectrometer and a method for skin-layer specific spectroscopy shall be proposed which allow for subcutaneous sampling via spectroscopic techniques, such as NIR spectroscopy.

### Summary

This problem is addressed by a spectrometer, a method for skin-layer specific spectroscopy, a computer program, a computer-readable storage medium and a non-transient computer-readable storage medium with the features of the independent claims. Advantageous embodiments which might be realized in an isolated fashion or in any arbitrary combinations are listed in the dependent claims as well as throughout the specification.

In a first aspect of the present invention, a spectrometer, specifically near infrared spectrometer, is disclosed. The spectrometer comprises:
- at least one illumination source configured for illuminating a sample of a subject with at least one illumination light beam having at least one wavelength in an optical spectral region, wherein the illumination source is configured for transmitting the illumination light beam to the sample along a direction of propagation;
- at least one detector sensitive to light in the optical spectral range, wherein the detector is configured for detecting light impinging on the detector under at least one direction of collection ;
wherein the illumination source and the detector are separated by a baseline, wherein the direction of collection and the direction of propagation of the illumination light beam form an angle of inclination α with α ≠ 0.

The term "spectrometer" as used herein is a broad term and is to be given its ordinary and customary meaning to a person of ordinary skill in the art and is not to be limited to a special or customized meaning. The term specifically may refer, without limitation, to an optical device configured for acquiring at least one item of spectral information on at least one object. Specifically, the at least one item of spectral information may refer to at least one optical property or optically measurable property which is determined as a function of a wavelength, for one or more different wavelengths. More specifically, the optical property or optically measurable property, as well as the at least one item of spectral information, may relate to at least one property characterizing at least one of a transmission, an absorption, a reflection and an emission of the at least one object, either by itself or after illumination with external light. The at least one optical property may be determined for one or more wavelengths. The spectrometer specifically may form an apparatus which is capable of recording a signal intensity with respect to the corresponding wavelength of a spectrum or a partition thereof, such as a wavelength interval, wherein the signal intensity may, specifically, be provided as an electrical signal which may be used for further evaluation. The spectrometer may specifically be a near infrared spectrometer, more specifically a device capable of recording a signal intensity at least in the near infrared spectral range. The near infrared spectrometer may specifically comprise an instrument configured for recording diffusive reflectance near infrared spectra.

The spectrometer, as an example, may be or may comprise a device which allows for a measurement of at least one spectrum, e.g. for the measurement of a spectral flux, specifically as a function of a wavelength or detection wavelength. The spectrum may be acquired, as an example, in absolute units or in relative units, e.g. in relation to at least one reference measurement. Thus, as an example, the acquisition of the at least one spectrum specifically may be performed either for a measurement of the spectral flux (unit W/nm) or for a measurement of a spectrum relative to at least one reference material (unit 1), which may describe the property of a material, e.g., reflectance over wavelength. Additionally or alternatively, the reference measurement may be based on a reference light source, an optical reference path, a calculated reference signal, e.g. a calculated reference signal from literature, and/or on a reference device.

Specifically, the at least one spectrometer may be a diffusive reflective spectrometer configured for acquiring spectral information from the light which is diffusively reflected by the at least one object, e.g. the at least one sample. Additionally or alternatively, the at least one spectrometer may be or may comprise an absorption- and/or transmission spectrometer. In particular, measuring a spectrum with the spectrometer may comprise measuring absorption in a transmission configuration. Specifically, the spectrometer may be configured for measuring absorption in a transmission configuration. As outlined above, however, other types of spectrometers are also feasible.

The at least one spectrometer, specifically and as will be outlined in further detail below, may comprise at least one illumination source which, as an example, may comprise at least one of a tunable light source, a light source having at least one fixed emission wavelength and a broadband light source. The spectrometer, as will be outlined in further detail below, further comprises at least one detector configured for detecting light, such as light which is at least one of transmitted, reflected or emitted from the at least one sample. The spectrometer further may comprise, as will be outlined in further detail below, at least one dispersive element, such as at least one of a grating, a prism and a filter, e.g. a length variable filter having varying transmission properties over its lateral extension. The dispersive element may be used for separating incident light into a spectrum of constituent wavelength signals whose respective intensities are determined by employing the detector, such as a detector having a detector array as described below in more detail. However, it may also be possible to use a narrowband light source for exciting the sample within a narrow optical spectral range.

The spectrometer, specifically, may be a portable spectrometer. The term "portable" as used herein is a broad term and is to be given its ordinary and customary meaning to a person of ordinary skill in the art and is not to be limited to a special or customized meaning. The term specifically may refer, without limitation, to the property of at least one object of being moved by human force, such as by a single user. Specifically, the object characterized by the term "portable" may have a weight not exceeding 10 kg, specifically not exceeding 5 kg, more specifically not exceeding 1 kg or even not exceeding 500 g. Additionally or alternatively, the dimensions of the object characterized by the term "portable" may be such that the object extends by no more than 0.3 m into any dimension, specifically by no more than 0.2 m into any dimension. The object, specifically, may have a volume of no more than 0.03 m³, specifically of no more than 0.01 m³, more specifically no more than 0.001 m³ or even no more than 500 mm³. In particular, as an example, the portable spectrometer may have dimensions of e.g. 10 mm by 10 mm by 5 mm. Specifically, the portable spectrometer may be part of a mobile device or may be attachable to a mobile device, such as a notebook computer, a tablet, a cell phone, such as a smart phone, a smartwatch and/or a wearable computer, also referred to as "wearable", e.g. a body borne computer such as a wrist band or a watch. In particular, a weight of the spectrometer, specifically the portable spectrometer, may be in the range from 1 g to 100 g, more specifically in the range from 1 g to 10 g.

The spectrometer may be configured for acquiring at least one item of spectral information on at least one object. The term "spectroscopic information", also referred to as "spectral information" or as "an item of spectral information", as used herein is a broad term and is to be given its ordinary and customary meaning to a person of ordinary skill in the art and is not to be limited to a special or customized meaning. The term specifically may refer, without limitation, to an item of information, e.g. on at least one object and/or radiation emitted by at least one object, characterizing at least one optical property of the object, more specifically at least one item of information characterizing, e.g. qualifying and/or quantifying, at least one of a transmission, an absorption, a reflection and an emission of the at least one object. As an example, the at least one item of spectral information may comprise at least one intensity information, e.g. information on an intensity of light being at least one of transmitted, absorbed, reflected or emitted by the sample, e.g. as a function of a wavelength or wavelength sub-range over one or more wavelengths, e.g. over a range of wavelengths. Specifically, the intensity information may correspond to or be derived from the signal intensity, specifically the electrical signal, recorded by the spectrometer device with respect to a wavelength or a range of wavelengths of the spectrum.

The spectrometer specifically may be configured for acquiring at least one spectrum or at least a part of a spectrum of detection light propagating from the sample to the spectrometer. The spectrum may describe the radiometric unit of spectral flux, e.g. given in units of watt per nanometer (W/nm), or other units, e.g. as a function of the wavelength of the detection light. Thus, the spectrum may describe the optical power of light, e.g. in the NIR spectral range, in a specific wavelength band. The spectrum may contain one or more optical variables as a function of the wavelength, e.g. the power spectral density, electric signals derived by optical measurements and the like. The spectrum may indicate, as an example, the power spectral density and/or the spectral flux of the sample, e.g. relative to a reference sample, such as a transmittance and/or a reflectance of the sample.

The spectrum, as an example, may comprise at least one measurable optical variable or property of the detection light and/or of the sample, specifically as a function of the illumination light and/or the detection light. As an example, the at least one measurable optical variable or property may comprise at least one at least one radiometric quantity, such as at least one of a spectral density, a power spectral density, a spectral flux, a radiant flux, a radiant intensity, a spectral radiant intensity, an irradiance, a spectral irradiance. Specifically, as an example, the spectrometer, specifically the detector, may measure the irradiance in Watt per square meter (W/m²), more specifically the spectral irradiance in Watt per square meter per nanometer (W/m²/nm). Based on the measured quantity the spectral flux in Watt per nanometer (W/nm) and/or the radiant flux in Watt (W) may be determined, e.g. calculated, by taking into account an area of the detector. The spectrum may specifically comprise a near infrared spectrum. The near infrared spectrum may be a diffusive reflectance near infrared spectrum given in units of absorbance *a* with *a* = -log₁₀(*reflectance*). The reflectance R may specifically be a background-corrected reflectance spectrum *R* = (*S* - *BG*) / (*R* - *BG*). The sample measurement *S* may be a raw detector signal over wavelength that is recorded from the sample of the subject with the spectrometer, specifically with the near infrared spectrometer. The reference measurement R may be a raw detector signal over wavelength that is recorded from a reference sample, such as a sample with known reflectance over wavelength, e.g. of 100% or 2%. The background measurement *BG* may be for a raw detector signal over wavelength of an instrument background that is recorded with the spectrometer, specifically with the near infrared spectrometer, using either an open port or a very dark reflectance target e.g. with reflectance of below 0.1%.

As outlined above, the spectrometer comprises the at least one illumination source configured for illuminating the sample of the subject with the at least one illumination light beam having at least one wavelength in the optical spectral region, wherein the illumination source is configured for transmitting the illumination light beam to the sample along the direction of propagation.

As used herein, the term "light" as used herein is a broad term and is to be given its ordinary and customary meaning to a person of ordinary skill in the art and is not to be limited to a special or customized meaning. The term specifically may refer, without limitation, to electromagnetic radiation in the optical spectral range. The term "optical spectral range" may refer, without limitation, to one or more of the infrared, the visible and the ultraviolet spectral range. As further used herein, the term "wavelength" may refer, without limitation to a distance between consecutive corresponding points of the same phase of the electromagnetic radiation. The optical spectral range may specifically comprise a skin-layer specific spectral range, specifically a near infrared spectral range.

Further, the term "ultraviolet spectral range", generally, refers to electromagnetic radiation having a wavelength of 1 nm to 380 nm, preferably of 100 nm to 380 nm. Further, in partial accordance with standard ISO-21348 in a valid version at the date of this document, the term "visible spectral range", generally, refers to a spectral range of 380 nm to 760 nm. The term "infrared spectral range" (IR) generally refers to electromagnetic radiation of 760 nm to 1000 µm, wherein the range of 760 nm to 1.5 µm is usually denominated as "near infrared spectral range" (NIR) while the range from 1.5 µm to 15 µm is denoted as "mid infrared spectral range" (MidIR) and the range from 15 µm to 1000 µm as "far infrared spectral range" (FIR). Preferably, light used for the typical purposes of the present invention is light in the infrared (IR) spectral range, more preferred, in the near infrared (NIR) and/or the mid infrared spectral range (MidIR), especially the light having a wavelength of 800 nm to 5 µm, preferably of 800 nm to 3 µm. This is due to the fact that many material properties or properties on the chemical constitution of many objects may be derived from the near infrared spectral range. It shall be noted, however, that spectroscopy in other spectral ranges is also feasible and within the scope of the present invention.

The term "illumination source" as used herein is a broad term and is to be given its ordinary and customary meaning to a person of ordinary skill in the art and is not to be limited to a special or customized meaning. The term specifically may refer, without limitation, to an arbitrary device configured for generating or providing light in the sense of the above-mentioned definition. The illumination source specifically may be or may comprise at least one electrical light source, such as an electrically driven light source. The illumination source may specifically be a broadband light source emitting light at least in the optical spectral range, specifically at least in the near infrared spectral range.

The term "illuminating" as used herein is a broad term and is to be given its ordinary and customary meaning to a person of ordinary skill in the art and is not to be limited to a special or customized meaning. The term specifically may refer, without limitation, to a process of exposing at least one sample to light. The process of exposing the sample to light may specifically comprise generating light and directing the generated light to the sample, such as by controlling a direction of propagation of the light, e.g. by generating a light beam. Thus, specifically, the illumination source may be configured for generating a light beam such that the generated light is directed to the sample of the subject.

The term "light beam" as used herein is a broad term and is to be given its ordinary and customary meaning to a person of ordinary skill in the art and is not to be limited to a special or customized meaning. The term specifically may refer, without limitation, to an amount of light emitted and/or reflected into a specific direction. The light beam may be a bundle of light rays having a predetermined extension in a direction perpendicular to a direction of propagation of the light beam. Preferably, the light beam may be or may comprise one or more Gaussian light beams, such as a linear combination of Gaussian light beams, which may be characterized by one or more Gaussian beam parameters, such as one or more of a beam waist, a Rayleigh-length or any other beam parameter or combination of beam parameters suited to characterize a development of a beam diameter and/or a beam propagation in space. As used herein, the term "ray" generally refers to a line that is perpendicular to wavefronts of lightand that points in a direction of energy flow. As used herein, the term "beam" generally refers to a collection of rays. In the following, the terms "ray" and "beam" will be used as synonyms. Thus, specifically, the term "light beam" generally refers to an amount of light, specifically an amount of light traveling essentially in the same direction, including the possibility of the light beam having a spreading angle or widening angle. The light beam may have a spatial extension. Specifically, the light beam may have a Gaussian beam profile. Consequently, the term "illumination light beam", as used herein, may refer, without limitation, to a light beam generated by the illumination source. The illumination light beam may specifically comprise a light beam propagating from the illumination source to the sample of the subject.

The term "direction of propagation" as used herein is a broad term and is to be given its ordinary and customary meaning to a person of ordinary skill in the art and is not to be limited to a special or customized meaning. The term specifically may refer, without limitation, to a line along which light is moving. Specifically, the direction of propagation may comprise a line along which the illumination light beam is moving towards the sample of the subject. Thus, in other words, the direction of propagation may comprise a line that is perpendicular to a wavefront of the illumination light, and that points in a direction of energy flow of the illumination light beam.

The term "subject" as used herein is a broad term and is to be given its ordinary and customary meaning to a person of ordinary skill in the art and is not to be limited to a special or customized meaning. The term specifically may refer, without limitation, to an arbitrary living object. The subject may be or may comprise one or more living beings and/or one or more parts thereof, such as one or more body parts of a human being, e.g. a user and/or an animal.

The term "sample" as used herein is a broad term and is to be given its ordinary and customary meaning to a person of ordinary skill in the art and is not to be limited to a special or customized meaning. The term specifically may refer, without limitation, to a part of the subject which may fully or partially be analyzed by spectroscopic methods. The sample may specifically refer to a sample that is investigated by the spectrometer or the method. For example, the sample of the subject may comprise a skin, more specifically a skin of a human being, e.g. of a user of the spectrometer. However, other samples are also feasible.

The illumination source may, as an example, comprise at least one light source configured for generating the illumination light beam. The illumination source may further comprise at least one optical sending fiber configured for transmitting the illumination light beam from the light source to the sample. Thus, in this example, the optical sending fiber may define a position of the illumination source with respect to the sample of the subject. The light source may comprise at least one light source selected from the group consisting of: an incandescent lamp; a light emitting diode (LED); a laser; a laser diode; a solid-state laser; a gas laser; a quantum cascade laser; a plasma light source; a gas discharge lamp, such as low pressure discharge lamps and/or high pressure lamps. For example, the light source may comprise at least one light-emitting diode and at least one luminescent material for light-conversion of primary light generated by the light-emitting diode. For example, the luminescent material may comprise phosphor. Thus, the light source may be or may comprise a phosphor LED.

The illumination light beam may have a wavelength from 800 nm to 3000 nm, preferably from 1250 nm to 2500 nm, more preferably from 1700 nm to 1800 nm and/or from 2200 nm to 2400 nm.

The optical spectral range may be a narrowband spectral range, specifically in the near infrared spectral range. The narrowband spectral range may comprise a wavelength interval of 500 nm, specifically of 200 nm, more specifically of 100 nm. The illumination source may be configured for generating the narrowband spectral range. As an example, the illumination source may comprise a narrowband light source, specifically being configured for emitting light in the narrowband spectral range.

Alternatively, the illumination light source may comprise a broadband light source. Thus, in this case, the spectrometer may comprise at least one dispersive element arranged in front of the detector in the direction of collection of a light beam from the sample to the detector. The term "dispersive element" as used herein is a broad term and is to be given its ordinary and customary meaning to a person of ordinary skill in the art and is not to be limited to a special or customized meaning. The term specifically may refer, without limitation, to an arbitrary optical element which interacts with differing spectral portions of incident light in a different manner, e.g. by having at least one wavelength-dependent optical property, such as at least one wavelength-dependent optical property selected from the list consisting of: a degree of reflection, a direction of reflection, a degree of refraction, a direction of refraction, an absorption, a transmission, an index of refraction. Specifically, the dispersive element may be an optical element configured for splitting light into different wavelengths, such as a grating, a filter or the like.

The dispersive element may specifically be selected from the group of a tunable dispersive element and a dispersive element having a fixed transmission spectrum. By using a tunable dispersive element, as an example, differing wavelength ranges may be selected sequentially, whereas, by using a dispersive element having a fixed transmission spectrum, the selection of the wavelength ranges may be fixed and may, however, be dependent e.g. on a detection position, thereby allowing for simultaneously exposing different detectors and/or different photosensitive elements of the detector to differing spectral ranges of light.

The at least one dispersive element may comprise at least one of a filter, a grating, a prism, a plasmonic filter, a diffractive optical element and a metamaterial. More specifically, the spectrometer may comprise at least one filter element disposed in a beam path of the light from the subject, i.e. in the beam path of the detection light, wherein the filter element, specifically may be configured such that each of the photosensitive elements is exposed to an individual spectral range of the light from the object. As an example, a variable filter element may be used, the transmission of which depends on a position on the filter element, such that, when the variable filter element is placed on top of the array of photosensitive elements, the individual photosensitive elements are exposed to differing spectral ranges of the incident light, specifically the detection light from the sample. Additionally or alternatively, the at least one dispersive element may comprise at least one of the following elements: an array of individual bandpass filters, an array of patterned filters, a MEMS-Interferometer, a MEMS-Fabry Perot interferometer. Further elements are feasible.

As further outlined above, the spectrometer comprises the at least one detector sensitive to light in the optical spectral range, wherein the detector is configured for detecting light impinging on the detector under the at least one direction of collection.

The term "detecting" as used herein is a broad term and is to be given its ordinary and customary meaning to a person of ordinary skill in the art and is not to be limited to a special or customized meaning. The term specifically may refer, without limitation, to a process of at least one of determining, measuring and monitoring at least one parameter, qualitatively and/or quantitatively, such as at least one of a physical parameter, a chemical parameter and a biological parameter. Specifically, the physical parameter may be or may comprise an electrical parameter. Consequently, the term "detector" as used herein is a broad term and is to be given its ordinary and customary meaning to a person of ordinary skill in the art and is not to be limited to a special or customized meaning. The term specifically may refer, without limitation, to an arbitrary device configured for detecting, i.e. for at least one of determining, measuring and monitoring, at least one parameter, qualitatively and/or quantitatively, such as at least one of a physical parameter, a chemical parameter and a biological parameter. The detector may be configured for generating at least one detector signal, more specifically at least one electrical detector signal, such as an analogue and/or a digital detector signal, the detector signal providing information on the at least one parameter measured by the detector. The detector signal may directly or indirectly be provided by the detector to an evaluation unit, such that the detector and the evaluation unit may be directly or indirectly connected. The detector signal may be used as a "raw" detector signal and/or may be processed or preprocessed before further used, e.g. by filtering and the like. Thus, the detector may comprise at least one processing device and/or at least one preprocessing device, such as at least one of an amplifier, an analogue/digital converter, an electrical filter and a Fourier transformation. The detector may specifically comprise a detector sensitive to near infrared light, e.g. comprising PbS or PbSe.

The detector may be or may comprise at least one optical detector. The optical detector may be configured for determining at least one optical parameter, such as an intensity and/or a power of light by which at least one sensitive area of the detector is irradiated. More specifically, the optical detector may comprise at least one photosensitive element and/or at least one optical sensor, such as at least one of a photodiode, a photocell, a photosensitive resistor, a phototransistor, a thermophile sensor, a photoacoustic sensor, a pyroelectric sensor, a photomultiplier and a bolometer. The detector, thus, may be configured for generating at least one detector signal, more specifically at least one electrical detector signal, in the above-mentioned sense, providing information on at least one optical parameter, such as the power and/or intensity of light by which the detector or a sensitive area of the detector is illuminated.

The detector may comprise one single optically sensitive element or area or a plurality of optically sensitive elements or areas. Specifically, the detector may be or may comprise at least one detector array, more specifically an array of photosensitive elements. Each of the photosensitive elements may comprise at least a photosensitive area which may be adapted for generating an electrical signal depending on the intensity of the incident light, wherein the electrical signal may, in particular, be provided to the evaluation unit, as will be outlined in further detail below.

The photosensitive area as comprised by each of the optically sensitive elements may, especially, be a single, uniform photosensitive area which is configured for receiving the incident light which impinges on the individual optically sensitive elements. However, other arrangements of the optically sensitive elements may also be conceivable.

The array of optically sensitive elements may be designed to generate detector signals, preferably electronic signals, associated with the intensity of the incident light which impinges on the individual optically sensitive elements. The detector signal may be an analogue and/or a digital signal. The electronic signals for adjacent pixelated sensors can, accordingly, be generated simultaneously or else in a temporally successive manner. By way of example, during a row scan or line scan, it is possible to generate a sequence of electronic signals which correspond to the series of the individual optically sensitive elements which are arranged in a line. In addition, the individual optically sensitive elements may, preferably, be active pixel sensors which may be adapted to amplify the electronic signals prior to providing it to the evaluation unit. For this purpose, the detector may comprise one or more signal processing devices, such as one or more filters and/or analogue-digital-converters for processing and/or preprocessing the electronic signals.

In case the detector comprises an array of optically sensitive elements, the detector, as an example, may be selected from any known pixel sensor, in particular, from a pixelated organic camera element, preferably, a pixelated organic camera chip, or from a pixelated inorganic camera element, preferably, a pixelated inorganic camera chip, more preferably from a CCD chip or a CMOS chip, which are, commonly, used in various cameras nowadays. As an alternative, the detector generally may be or comprise a photoconductor, in particular an inorganic photoconductor, especially PbS, PbSe, Ge, InGaAs, ext. InGaAs, InSb, or HgCdTe. As a further alternative it may comprise at least one of pyroelectric, bolometer or thermophile detector elements. Thus, a camera chip having a matrix of 1 x N pixels or of M x N pixels may be used here, wherein, as an example, M may be < 10 and N may be in the range from 1 to 50, preferably from 2 to 20, more preferred from 5 to 10. Further, a monochrome camera element, preferably a monochrome camera chip, may be used, wherein the monochrome camera element may be differently selected for each optically sensitive element, especially, in accordance with the varying wavelength along the series of the optical sensors.

Thus, the array may be adapted to provide a plurality of the electrical signals which may be generated by the photosensitive areas of the optically sensitive elements comprised by the array. The electrical signals as provided by the array of the spectrometer device may be forwarded to the evaluation unit.

Alternatively or additionally, the detector may comprise at least one photosensitive element configured for generating at least one detector signal dependent on an illumination of its light-sensitive region with detection light. The detector may comprise an array of photosensitive elements, wherein each of the photosensitive elements may be configured for generating at least one detector signal dependent on an illumination of its light-sensitive region with detection light. The spectrometer may specifically be configured such that the photosensitive elements are sensitive to differing spectral ranges of the detection light. The photosensitive element may comprise at least one photoconductive material selected from the group consisting of lead sulfide (PbS); lead selenide (PbSe); mercury cadmium telluride (HgCdTe); cadmium sulfide (CdS); cadmium selenide (CdSe); indium antimonide (InSb); indium arsenide (InAs); indium gallium arsenide (InGaAs); silicon (Si); silicon germanium (SiGe); an extrinsic semiconductor; an organic semiconductor.

The term "direction of collection" as used herein is a broad term and is to be given its ordinary and customary meaning to a person of ordinary skill in the art and is not to be limited to a special or customized meaning. The term specifically may refer, without limitation, to a line along which light is travelling from the sample to the detector. Specifically, the direction of collection may define a line along which the detection light is travelling from the sample to the detector. The direction of collection may comprise a viewing direction of the detector, such as a direction along which the detector is viewing at the sample of the subject. The direction of collection may also be referred to as a direction of readout.

Further, as outlined above, the illumination source and the detector are separated by the baseline, wherein the direction of collection and the direction of propagation of the illumination light beam form the angle of inclination α with α ≠ 0.

The term "baseline" as used herein is a broad term and is to be given its ordinary and customary meaning to a person of ordinary skill in the art and is not to be limited to a special or customized meaning. The term specifically may refer, without limitation, to a distance between the illumination source and a light detection spot. The baseline may specifically be a distance in a plane defined by the sample of the subject. The baseline may be a distance between a position where the illumination light beam propagating from the illumination source to the sample of the subject impinges on the sample of the subject and a light detection spot, such as an area on the sample of the subject from where light is collected for being detected by the detector. The baseline may be defined between a centroid of the position where the illumination light beam impinges on the sample of the subject and the light detection spot. The baseline may specifically be from 1 to 5 mm, preferably the baseline is ≥ 3 mm.

The term "angle of inclination" as used herein is a broad term and is to be given its ordinary and customary meaning to a person of ordinary skill in the art and is not to be limited to a special or customized meaning. The term specifically may refer, without limitation, to the acute angle defined by the direction of propagation of the illumination beam and the direction of collection.

Since the illumination source and the detector are separated by the baseline, the angle of inclination may be defined by parallel a projection of the direction of the illumination beam towards the direction of collection, or vice versa. The angle of inclination α may be in the range of - 85° ≤ α < 0°, preferably -85° ≤ α ≤ -50°. The negative range of the angle of inclination refers to a viewing direction of the detector facing away from the direction of propagation of the illumination light beam, in particular such that the direction of collection is opposite rather than towards to the direction of propagation. As an example, the baseline may be ≥ 3 mm and the angle of inclination α may be in the range of -85° ≤ α ≤ -50°.

An aperture of the illumination source and an aperture of the detector may be tilted with respect to each other. Alternatively or additionally, the illumination source and/or the detector may comprise at least one lens. The spectrometer may also comprise a combination of apertures and lens for the illumination source and the detector. Thus, specifically, the detector may comprise a combination of an aperture and a lens. Optionally, the illumination source may also comprise at least one of an aperture and a lens. A numerical aperture of the detector may be in the range of 0.1 ≤ NA ≤ 0.5, preferably in the range of 0.22 ≤ NA ≤ 0.39. The combination of apertures of the illumination source and the detector and/or the combination of aperture and lens for the illumination source and/or the detector may enable the separation of the illumination source and the detector by the baseline and the angle of inclination between the direction of collection and the direction of propagation of the illumination light beam.

Alternatively or additionally, the detector may comprise at least one optical receiving fiber configured for receiving at least one incident light beam generated by the sample upon interaction with the illumination light beam, and optionally transferring the light beam to the dispersive element. Thus, specifically, the optical receiving fiber may define a light detection spot at the sample and the direction of collection for the detection light and may be configured be guiding the light to the detector. The dispersive element may be arranged in front of the detector such that the detection light traveling from the sample to the detector may pass the dispersive element prior to the detector.

The spectrometer may further comprise at least one evaluation device configured for evaluating the light detected by the detector, thereby determining at least one biomarker. The term "to evaluate", as used herein, is a broad term and is to be given its ordinary and customary meaning to a person of ordinary skill in the art and is not to be limited to a special or customized meaning. The term specifically may refer, without limitation, to the process of processing at least one first item of information in order to generate at least one second item of information thereby. Consequently, the term "evaluation unit", as used herein, is a broad term and is to be given its ordinary and customary meaning to a person of ordinary skill in the art and is not to be limited to a special or customized meaning. The term specifically may refer, without limitation, to an arbitrary device or a combination of devices configured to evaluate or process at least one first item of information, in order to generate at least one second item of information thereof. Thus, specifically, the evaluation unit may be configured for processing at least one input signal and to generate at least one output signal thereof. The at least one input signal, as an example, may comprise at least one of the first and second detector signals provided directly or indirectly by the at least one detector.

As an example, the evaluation unit may be or may comprise one or more integrated circuits, such as one or more application-specific integrated circuits (ASICs), and/or one or more data processing devices, such as one or more of computers, digital signal processors (DSP), field programmable gate arrays (FPGA) preferably one or more microcomputers and/or microcontrollers. Additional components may be comprised, such as one or more preprocessing devices and/or data acquisition devices, such as one or more devices for receiving and/or preprocessing of the detector signals, such as one or more AD-converters and/or one or more filters. Further, the evaluation unit may comprise one or more data storage devices. Further, the evaluation unit may comprise one or more interfaces, such as one or more wireless interfaces and/or one or more wire-bound interfaces.

The evaluation device may be configured, such as by software programming and/or by hardware means, for evaluating the detector signal to derive at least one item of spectral information on a subcutaneous region of the sample. The evaluation device may use a reference signal for the evaluation. The reference signal may specifically be or may comprise a spectral background. The spectral background may be a spectrum, specifically a near infrared spectrum, of the sample of the subject, e.g. of the skin, when the relevant biomarker is absent. Thus, specifically, the reference signal may be used to determine an absorbance contrast. The absorbance contrast may be a difference between the absorbance at two or more different wavelengths. The biomarker may be determined based on the item of spectral information on a subcutaneous region of the sample, such as the absorbance contrast. The biomarker may be at least one biomarker selected from the group consisting of: body temperature, body hydration, hemoglobin oxygenation, concentration of glucose, concentration of lactate, concentration of ethanol, concentration of metabolic products of ethanol, e.g. aldehyde, collagen level, skin hydration, sebum (fat) concentration. The biomarkers may specifically be evaluated in a chemometric approach, such as by using multivariate data analysis together with biomarker information from reference methods, e.g., the analysis of blood samples. Alternatively or additionally, classification or quantification algorithms can be applied, e.g., principle component analysis, least square regression, multiple linear regression.

The biomarker may specifically comprise at least one of a body biomarker and a skin biomarker. The body biomarker may be at least one measurable substance, characteristic, and/or molecule concentration in the body of the subject serving as an indicator of normal or disturbed biological processes in the body, specifically in the human body. The body biomarker may comprise at least one biomarker selected from the group consisting of: body temperature, body hydration, hemoglobin oxygenation, concentration of glucose, concentration of lactate, concentration of ethanol, metabolic products of ethanol, e.g., aldehyde, collagen level, skin hydration, sebum (fat) concentration. The skin biomarker may be a body biomarker that can be measured at the skin surface or through the skin of the subject. The skin biomarker may comprise at least one biomarker selected from the group consisting of: a concentration of an abundant molecule, e.g., water, glucose, lactate, or ethanol. For example, the biomarker may comprise a blood alcohol concentration. The blood alcohol concentration may be a concentration of ethanol in the blood of the subject given in units volume per volume (‰).

In a further aspect of the present invention, a method for skin-layer specific spectroscopy, specifically for skin-layer specific near infrared spectroscopy, is disclosed. The method comprises using at least one spectrometer according to the present invention, such as according to any one of the embodiments disclosed above and/or according to any one of the embodiments disclosed in further detail below. Thus, for definitions of terms and/or possible embodiments of the method, reference is made to the description of the spectrometer above. The method may specifically comprise using a near infrared spectrometer.

The method comprises the following steps that may be performed in the given order. However, a different order may also be possible. In particular, one, more than one or even all of the method steps may be performed once or repeatedly. Further, the method steps may be performed successively or, alternatively, one or more of the method steps may be performed in a timely overlapping fashion or even in a parallel fashion and/or in a combined fashion. The method may further comprise additional method steps that are not listed.

The method comprises the following steps:
a. illuminating a sample of a subject with at least one illumination light beam having at least one wavelength in an optical spectral range, specifically in a near infrared spectral range, by using the illumination source, wherein the illumination light beam is transmitted to the sample along a direction of propagation;
b. detecting light, generated by the sample upon interaction with the illumination light beam, impinging on the detector under at least one direction of collection;
c. evaluating the detected light to derive at least one item of spectral information on a subcutaneous region of the sample.

The sample of the subject may comprise a skin sample. For example, the sample of the subject may comprise a skin sample of a living being, such as from a human being, e.g. from a user of the spectrometer, from another person and/or from an animal.

The method may be at least one method selected from the group consisting of: an in-vivo method; an ex-vivo method; an in-vitro method. The in-vivo method may specifically comprise a measurement of biomarker, specifically of body or skin biomarkers, on a living organism, e.g. on an animal or a human being. The ex-vivo method may specifically comprise a measurement of biomarker, specifically of body or skin biomarkers, on tissue of actual but not living organism. The in-vitro method may specifically comprise a measurement or experiment conducted outside of a living organism, typically in a controlled laboratory environment, using isolated cells or tissue or extracted tissue from an animal or human tissue. Alternatively or additionally, the method may also be performed on a skin phantom, such as on a system that imitates properties of human skin in terms of skin's NIR spectrum. In particular, the skin phantom may be configured for mimicking NIR absorption and scattering properties of real skin.

Alternatively or additionally, the item of spectral information on the subcutaneous region of the sample may be used for determining at least one biomarker, wherein the biomarker is at least one biomarker selected from the group consisting of: body temperature, body hydration, hemoglobin oxygenation, concentration of glucose, concentration of lactate, concentration of ethanol, concentration of metabolic products of ethanol, e.g., aldehyde, collagen level, skin hydration, sebum (fat) concentration. The biomarkers may specifically be evaluated in a chemometric approach, such as by using multivariate data analysis together with biomarker information from reference methods, e.g., the analysis of blood samples. Alternatively or additionally, classification or quantification algorithms can be applied, e.g., principle component analysis, least square regression, multiple linear regression.

Alternatively or additionally, the method specifically may be performed on-line, e.g. in the field. The spectrometer, specifically, may be a portable spectrometer which specifically may be used in the field. In particular, the spectrometer may be part of a mobile device or may be attachable to a mobile device, such as a notebook computer, a tablet, a cell phone, such as a smart phone, a smartwatch and/or a wearable computer, also referred to as "wearable", e.g. a body borne computer.

The method may fully or partially be computer-implemented. The term "computer-implemented" as used herein, is a broad term and is to be given its ordinary and customary meaning to a person of ordinary skill in the art and is not to be limited to a special or customized meaning. The term specifically may refer, without limitation, to a method involving at least one computer and/or at least one computer network. The computer and/or computer network may comprise at least one processor which is configured for performing at least one of the method steps of the method according to the present invention. Preferably, each of the method steps is performed and/or controlled by the computer and/or computer network. The method may be performed completely automatically, specifically without user interaction. Thus, at least step c. of the method may be computer-implemented.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

In a further aspect of the present invention, a computer program is disclosed, comprising instructions which, when the program is executed by the spectrometer according to the present invention, such as according to any one of the embodiments disclosed above and/or according to any one of the embodiments disclosed in further detail below, cause the spectrometer to perform the method according to the present invention, such as according to any one of the embodiments disclosed above and/or according to any one of the embodiments disclosed in further detail below.

In a further aspect of the present invention, a computer-readable storage medium is disclosed, comprising instructions which, when the instructions are executed by the spectrometer according to the present invention, such as according to any one of the embodiments disclosed above and/or according to any one of the embodiments disclosed in further detail below, cause the spectrometer to perform the method according to the present invention, such as according to any one of the embodiments disclosed above and/or according to any one of the embodiments disclosed in further detail below.

As used herein, the term "computer-readable storage medium" specifically may refer to non-transitory data storage means, such as a hardware storage medium having stored thereon computer-executable instructions. The computer-readable storage medium specifically may be or may comprise a storage medium such as a random-access memory (RAM) and/or a read-only memory (ROM). The computer-readable storage medium may also be referred to as "computer-readable data carrier".

In a further aspect of the present invention, a non-transient computer-readable medium is disclosed, including instructions that, when executed by one or more processors, cause the one or more processors to perform the method according to the present invention, such as according to any one of the embodiments disclosed above and/or according to any one of the embodiments disclosed in further detail below.

In a further aspect of the present invention, a use of a spectrometer according to the present invention, such as according to any one of the embodiments disclosed above and/or according to any one of the embodiments disclosed in further detail below, is disclosed, for skin-layer specific near infrared spectroscopy.

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically are used only once when introducing the respective feature or element. In most cases, when referring to the respective feature or element, the expressions "at least one" or "one or more" are not repeated, nonwithstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The spectrometer and the method according to the present invention may provide a large number of advantages over known methods and devices of similar kind. Specifically, the spectrometer and the method according to the present invention may provide means for subcutaneous sampling via spectroscopic techniques, such as NIR spectroscopy. It has been shown that a spatial separation of illumination source and detector enables to sample deeper skin layers, e.g. dermis sampling. Previously, no selection of the light collection angle has been reported. The present invention may combine both illumination source-detector spacing as well as the variation of direction of readout. The angle of inclination between the direction of collection and the direction of propagation may be obtained by a combination of apertures or a combination of aperture and lens in the read-out path of the spectrometer. Preferably, a spacing of > 3 mm and an angle of inclination of < -50° may be particularly advantageously since almost the complete information of about 80-95 % of light can be collected from the subcutaneous layer. The negative angle of inclination may refer to a detector path that is looking away from the illumination light source. The spectrometer and the method according to the present invention may specifically provide subcutaneous sample without the need for reference measurements or corrections of epidermis or dermis sampling.

Summarizing and without excluding further possible embodiments, the following embodiments may be envisaged:
Embodiment 1: A spectrometer, specifically near infrared spectrometer, comprising
   - at least one illumination source configured for illuminating a sample of a subject with at least one illumination light beam having at least one wavelength in an optical spectral range, wherein the illumination source is configured for transmitting the illumination light beam to the sample along a direction of propagation;
   - at least one detector sensitive to light in the optical spectral range, wherein the detector is configured for detecting light impinging on the detector under at least one direction of collection;
   wherein the illumination source and the detector are separated by a baseline, wherein the direction of collection and the direction of propagation of the illumination light beam form an angle of inclination α with α ≠ 0.
Embodiment 2: The spectrometer according to the preceding embodiment, wherein the angle of inclination α is in the range of -85° ≤ α < 0°, preferably -85° ≤ α ≤ -50°.
Embodiment 3: The spectrometer according to the preceding embodiment, wherein the negative range of the angle of inclination refers to a viewing direction of the detector facing away from the direction of propagation of the illumination light beam.
Embodiment 4: The spectrometer according to any one of the preceding embodiments, wherein the baseline is from 1 to 5 mm, preferably the baseline is ≥ 3 mm.
Embodiment 5: The spectrometer according to any one of the preceding embodiments, wherein an aperture of the illumination source and an aperture of the detector are tilted with respect to each other.
Embodiment 6: The spectrometer according to any one of the preceding embodiments, wherein a numerical aperture of the detector is in the range of 0.1 ≤ NA ≤ 0.5, preferably in the range of 0.22 ≤ NA ≤ 0.39.
Embodiment 7: The spectrometer according to any one of the preceding embodiments, wherein the illumination source and/or the detector comprises at least one lens.
Embodiment 8: The spectrometer according to any one of the preceding embodiments, wherein the illumination source comprises at least one light source configured for generating the illumination light beam, wherein the illumination source comprises at least one optical sending fiber configured for transmitting the illumination light beam from the light source to the sample.
Embodiment 9: The spectrometer according to the preceding embodiment, wherein the light source comprises at least one light source selected from the group consisting of: an incandescent lamp; a light emitting diode (LED); a laser; a laser diode; a solid-state laser; a gas laser; a quantum cascade laser; a plasma light source; a gas discharge lamp, such as low pressure discharge lamps and/or high pressure lamps.
Embodiment 10: The spectrometer according to any one of the two preceding embodiments, wherein the light source comprising at least one light-emitting diode and at least one luminescent material for light-conversion of primary light generated by the light-emitting diode.
Embodiment 11: The spectrometer according to any one of the preceding embodiments, wherein the optical spectral range is a narrowband spectral range, specifically in the near infrared spectral range.
Embodiment 12: The spectrometer according to the preceding embodiment, wherein the illumination source is configured for generating the narrowband spectral range.
Embodiment 13: The spectrometer according to the preceding embodiment, wherein the illumination source comprises a narrowband light source.
Embodiment 14: The spectrometer according to any one of the preceding embodiments, further comprising at least one dispersive element arranged in front of the detector in the direction of collection of a light beam from the sample to the detector.
Embodiment 15: The spectrometer according to the preceding embodiment, wherein the dispersive element is selected from the group of a tunable dispersive element and a dispersive element having a fixed transmission spectrum.
Embodiment 16: The spectrometer according to any one of the preceding embodiments, wherein the detector comprises at least one optical receiving fiber configured for receiving at least one incident light beam generated by the sample upon interaction with the illumination light beam, and optionally transferring the light beam to a dispersive element.
Embodiment 17: The spectrometer according to any one of the preceding embodiments, wherein the illumination light beam has a wavelength from 800 nm to 3000 nm, preferably from 1250 nm to 2500 nm, more preferably from 1700 nm to 1800 nm and/or from 2200 nm to 2400 nm.
Embodiment 18: The spectrometer according to any one of the preceding embodiments, wherein the optical spectral range comprises a skin-layer specific spectral range, specifically a near infrared spectral range.
Embodiment 19: The spectrometer according to any one of the preceding embodiments, wherein the spectrometer comprises at least one evaluation device configured for evaluating the light detected by the detector, thereby determining at least one biomarker, wherein the biomarker is at least one biomarker selected from the group consisting of: body temperature, body hydration, hemoglobin oxygenation, concentration of glucose, concentration of lactate, concentration of ethanol, concentration of metabolic products of ethanol, e.g. aldehyde, collagen level, skin hydration, sebum (fat) concentration.
Embodiment 20: The spectrometer according to any one of the preceding embodiments, wherein the detector comprises at least one photosensitive element configured for generating at least one detector signal dependent on an illumination of its light-sensitive region with detection light.
Embodiment 21: The spectrometer according to any one of the preceding embodiments, wherein the detector comprises an array of photosensitive elements, wherein each of the photosensitive elements is configured for generating at least one detector signal dependent on an illumination of its light-sensitive region with detection light.
Embodiment 22: The spectrometer according to the preceding embodiment, wherein the spectrometer is configured such that the photosensitive elements are sensitive to differing spectral ranges of the detection light.
Embodiment 23: The spectrometer according to any one of the two preceding embodiments, wherein the photosensitive element comprises at least one photoconductive material selected from the group consisting of lead sulfide (PbS); lead selenide (PbSe); mercury cadmium telluride (HgCdTe); cadmium sulfide (CdS); cadmium selenide (CdSe); indium antimonide (InSb); indium arsenide (InAs); indium gallium arsenide (InGaAs); silicon (Si); silicon germanium (SiGe); an extrinsic semiconductor; an organic semiconductor.
Embodiment 24: A method for skin-layer specific spectroscopy, specifically for skin-layer specific near infrared spectroscopy, using at least one spectrometer according to any one of the preceding embodiments, specifically a near infrared spectrometer, comprising the following steps:
   a. illuminating a sample of a subject with at least one illumination light beam having at least one wavelength in an optical spectral range, specifically in a near infrared spectral range, by using the illumination source, wherein the illumination light beam is transmitted to the sample along a direction of propagation;
   b. detecting light, generated by the sample upon interaction with the illumination light beam, impinging on the detector under at least one direction of collection;
   c. evaluating the detected light to derive at least one item of spectral information on a subcutaneous region of the sample.
Embodiment 25: The method according to the preceding embodiment, wherein the sample of the subject comprises a skin sample.
Embodiment 26: The method according to any one of the two preceding embodiments, wherein the item of spectral information on the subcutaneous region of the sample is used for determining at least one biomarker, wherein the biomarker is at least one biomarker selected from the group consisting of: body temperature, body hydration, hemoglobin oxygenation, concentration of glucose, concentration of lactate, concentration of ethanol, concentration of metabolic products of ethanol, e.g., aldehyde, collagen level, skin hydration, sebum (fat) concentration.
Embodiment 27: A computer program comprising instructions which, when the program is executed by the spectrometer according to any one of the preceding embodiments referring to a spectrometer, cause the spectrometer to perform the method according to any one of the preceding embodiments referring to a method.
Embodiment 28: A computer-readable storage medium comprising instructions which, when the instructions are executed by the spectrometer according to any one of the preceding embodiments referring to a spectrometer cause the spectrometer to perform the method according to any one of the preceding embodiments referring to a method.
Embodiment 29: A non-transient computer-readable medium including instructions that, when executed by one or more processors, cause the one or more processors to perform the method according to any one of the preceding embodiments referring to a method.
Embodiment 30: A use of a spectrometer according to any one of the preceding embodiments referring to a spectrometer for skin-layer specific near infrared spectroscopy.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1A: shows an embodiment of a spectrometer according to the present invention in a schematic view;
- Figure 1B: shows a detailed view on the arrangement of the illumination source and the detector;
- Figures 2A to 2C: show simulation data of skin layer sampling; and
- Figure 3: shows a flow chart of an embodiment of a method for skin-layer specific spectroscopy.

### Detailed description of the embodiments

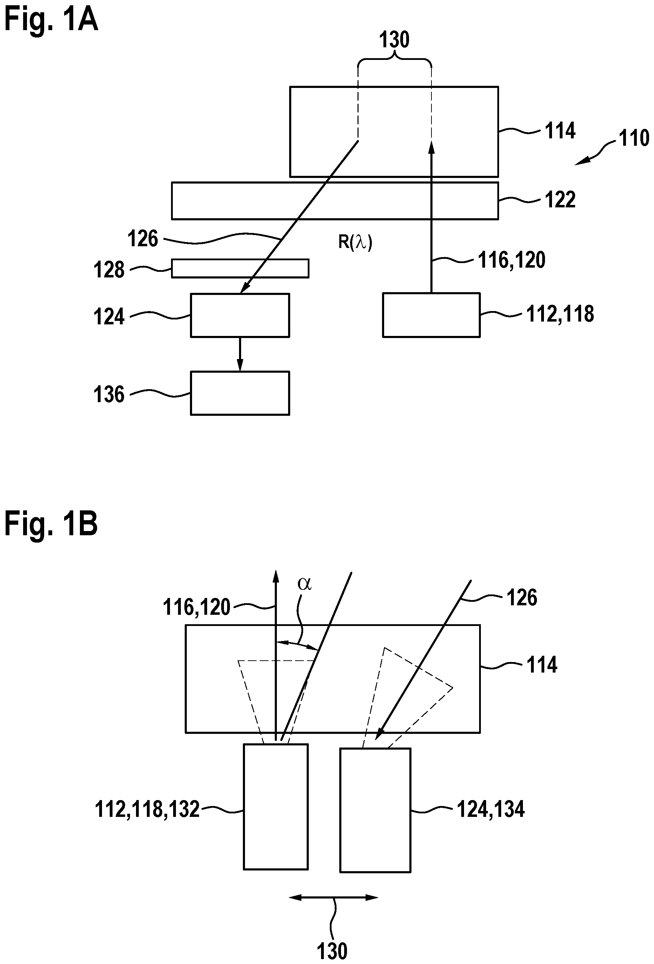

Figure 1 shows an embodiment of a spectrometer 110 according to the present invention in a schematic view. The spectrometer 110 may specifically be a near infrared spectrometer and, thus, may be configured for recording diffusive reflectance near infrared spectra. However, other spectrometers 110 are also feasible.

The spectrometer 110 comprises at least one illumination source 112 for illuminating a sample 114 of a subject with at least one illumination light beam 116 having at least one wavelength in an optical spectral range. For example, the sample 114 of the subject may be a skin of the user of the spectrometer 110. The illumination light beam 116 may have a wavelength from 800 nm to 3000 nm, preferably from 1250 nm to 2500 nm, more preferably from 1700 nm to 1800 nm and/or from 2200 nm to 2400 nm. Thus, preferably, the optical spectral range may be a skin-layer specific spectral range, specifically a near infrared spectral range. For example, the illumination source 112 may comprise at least one light source 118 comprising at least one light-emitting diode and at least one luminescent material for light-conversion of primary light generated by the light-emitting diode. The LED, via the luminescent material, may be configured for emitting light in a wavelength range from 800 nm to 3000 nm. Further, the illumination source 112 is configured for transmitting the illumination light beam 116 to the sample 114 along a direction of propagation 120.

Further, as shown in the exemplary embodiment of Figure 1, the spectrometer 110 may comprise at least one sample interface 122, such as at least one window being made from at least one transparent material configured for transmitting the illumination light beam 116 to the sample 114 of the subject.

The spectrometer 110 further comprises at least one detector 124 sensitive to light in the optical spectral range. The detector 124 is configured for detecting light impinging on the detector 124 under at least one direction of collection 126.

As an example, the detector 124 may comprise at least one photosensitive element configured for generating at least one detector signal dependent on an illumination of its light-sensitive region with detection light. The detector 124 may comprise an array of photosensitive elements, wherein each of the photosensitive elements may be configured for generating at least one detector signal dependent on an illumination of its light-sensitive region with detection light. The spectrometer 110 may specifically be configured such that the photosensitive elements are sensitive to differing spectral ranges of the detection light. The photosensitive element may comprise at least one photoconductive material selected from the group consisting of lead sulfide (PbS); lead selenide (PbSe); mercury cadmium telluride (HgCdTe); cadmium sulfide (CdS); cadmium selenide (CdSe); indium antimonide (InSb); indium arsenide (InAs); indium gallium arsenide (InGaAs); silicon (Si); silicon germanium (SiGe); an extrinsic semiconductor; an organic semiconductor.

Further, in this example, the spectrometer 110 may comprise at least one dispersive element 128 arranged in front of the detector 124 in the direction of collection 126 of a light beam from the sample 114 to the detector 124. The dispersive element 128 may e.g. comprise a variable filter element, the transmission of which depends on a position on the filter element, such that, when the variable filter element is placed on top of the array of photosensitive elements, the individual photosensitive elements are exposed to differing spectral ranges of the incident light, specifically the detection light from the sample 114. Other examples are also feasible. Further, as an alternatively or additionally to the dispersive element 128, it may also be possible to use a narrowband light source for exciting the sample 114 within a narrow optical spectral range.

As can be seen in Figure 1A, the illumination source 112 and the detector 124 are separated by a baseline 130. The baseline 130, as an example, may specifically be from 1 to 5 mm, preferably the baseline 130 is ≥ 3 mm.

Further, as can be seen best in Figure 1B showing a detailed view on the arrangement of the illumination source 112 and the detector 124, the direction of collection 126 and the direction of propagation 120 of the illumination light beam 116 form an angle of inclination α with α ≠ 0. As indicated in Figure 1B, the angle of inclination α can be determined from a projection of the direction of collection 126 to the direction of propagation 120 of the illumination light beam 116, or vice versa. The angle of inclination α may be in the range of - 85° ≤ α < 0°, preferably -85° ≤ α ≤ -50°. In this example, the baseline 130 may be ≥ 3 mm and the angle of inclination α may be in the range of - 85° ≤ α ≤ -50°. The negative range of the angle of inclination may refer to a viewing direction of the detector 124 facing away from the direction of propagation 120 of the illumination light beam 116. A spreading angle of the illumination light beam 116 may be in the range of 5° to 45°, specifically in the range of 10° to 25°. Similarly, the detection light traveling to the detector 124 may also form a light beam having a spreading angle in the range of 5° to 45°, specifically in the range of 10° to 25°.

As an example, the illumination source 112 may further comprise at least one optical sending fiber 132 configured for transmitting the illumination light beam 116 from the light source 118 to the sample 114. The optical sending fiber 132 may define a position of the illumination source 112 with respect to the sample 114 of the subject. Further, the detector 124 may comprise at least one optical receiving fiber 134 configured for receiving at least one incident light beam generated by the sample 114 upon interaction with the illumination light beam 116, and optionally transferring the light beam to the dispersive element 128. Thus, specifically, the optical receiving fiber 134 may define a light detection spot at the sample 114 and the direction of collection 126 for the detection light and may be configured be guiding the light to the detector 124. The dispersive element 128 may be arranged in front of the detector 124 such that the detection light traveling from the sample 114 to the detector 124 may pass the dispersive element 128 prior to the detector 124. Alternatively or additionally, the detector 124 may comprise a combination of an aperture and a lens (not shown in Figure 1B). The combination of aperture and lens for the detector 124 may enable the separation of the illumination source 112 and the detector 124 by the baseline 130 and the angle of inclination between the direction of collection 126 and the direction of propagation 120 of the illumination light beam 116.

Turning back to Figure 1A, the spectrometer 110 may further comprise at least one evaluation device 136 configured for evaluating the light detected by the detector 124, thereby determining at least one biomarker. The evaluation device 136 may be configured, such as by software programming and/or by hardware means, for evaluating the detector signal to derive at least one item of spectral information on a subcutaneous region of the sample 114. The evaluation device 136 may use a reference signal for the evaluation. The reference signal may specifically be or may comprise a spectral background. The spectral background may be a spectrum, specifically a near infrared spectrum, of the sample 114 of the subject, e.g. of the skin, when the relevant biomarker is absent. Thus, specifically, the reference signal may be used to determine an absorbance contrast. The absorbance contrast may be a difference between the absorbance at two or more different wavelengths. The biomarker may be determined based on the item of spectral information on a subcutaneous region of the sample 114, such as the absorbance contrast. The biomarker may be at least one biomarker selected from the group consisting of: body temperature, body hydration, hemoglobin oxygenation, concentration of glucose, concentration of lactate, concentration of ethanol, concentration of metabolic products of ethanol, e.g. aldehyde, collagen level, skin hydration, sebum (fat) concentration. The biomarkers may specifically be evaluated in a chemometric approach, such as by using multivariate data analysis together with biomarker information from reference methods, e.g., the analysis of blood samples. Alternatively or additionally, classification or quantification algorithms can be applied, e.g., principle component analysis, least square regression, multiple linear regression.

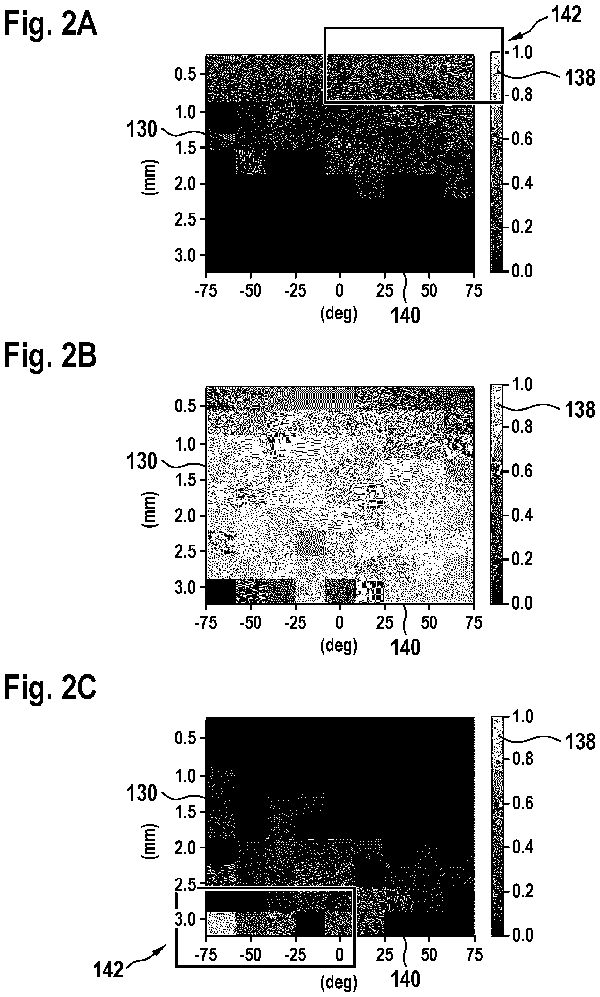

Figures 2A to 2C show simulation data of skin layer sampling. Specifically, Figure 2A shows simulation data for epidermis sampling, Figure 2B shows simulation data for dermis sampling and Figure 2C shows simulation data for subcutaneous sampling. Figures 2A to 2C specifically show heat maps of a fraction of an output power 138 of the detection light with respect to an input power of the illumination light beam 116 as a function of the baseline 130 in mm and the angle of inclination 140 in degree. The simulation was performed with a spreading angle of the illumination light beam 116 equal to a spreading angle of the detection light beam of 17.4°. Thus, these settings correspond to a numerical aperture of NA=0.22. As can be seen in Figures 2A and 2C, the simulation data indicate region-specific sampling intervals 142. Specifically, the interval of the baseline 130 of ≥ 3 mm and the angle of inclination α of - 85° ≤ α < 0°, preferably of - 85° ≤ α ≤ -50°, may be suitable for subcutaneous sampling. In this region, almost the complete information of about 80-95 % of light can be collected from the subcutaneous layer.

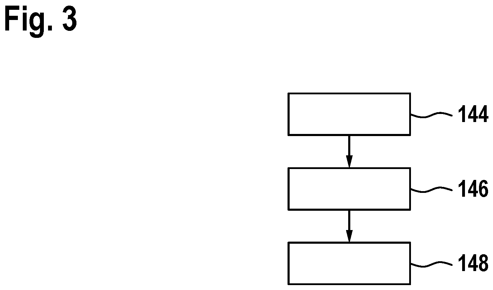

Figure 3 shows a flow chart of an embodiment of a method for skin-layer specific spectroscopy. The method comprises using at least one spectrometer 110 according to the present invention, such as according to the exemplary embodiment of Figures 1A and 1B and/or according to any other embodiment disclosed herein. Thus, for a detailed description of the spectrometer 110 used in the method, reference is made to the description Figures 1A and 1B above.

The method comprises the following steps that may be performed in the given order. However, a different order may also be possible. In particular, one, more than one or even all of the method steps may be performed once or repeatedly. Further, the method steps may be performed successively or, alternatively, one or more of the method steps may be performed in a timely overlapping fashion or even in a parallel fashion and/or in a combined fashion. The method may further comprise additional method steps that are not listed.

The method comprises the following steps:
a. (denoting by reference number 144) illuminating a sample 114 of a subject with at least one illumination light beam 116 having at least one wavelength in an optical spectral range, specifically in a near infrared spectral range, by using the illumination source 112, wherein the illumination light beam 116 is transmitted to the sample 114 along a direction of propagation 120;
b. (denoted by reference number 146) detecting light, generated by the sample 114 upon interaction with the illumination light beam 116, impinging on the detector 124 under at least one direction of collection 126;
c. (denoted by reference number 148) evaluating the detected light to derive at least one item of spectral information on a subcutaneous region of the sample 114.

The sample 112 of the subject may comprise a skin sample. For example, the sample 114 of the subject may comprise a skin sample of a living being, such as from a human being, e.g. from a user of the spectrometer 110, from another person and/or from an animal.

The item of spectral information on the subcutaneous region of the sample 114 may be used for determining at least one biomarker, wherein the biomarker is at least one biomarker selected from the group consisting of: body temperature, body hydration, hemoglobin oxygenation, concentration of glucose, concentration of lactate, concentration of ethanol, concentration of metabolic products of ethanol, e.g., aldehyde, collagen level, skin hydration, sebum (fat) concentration. The biomarkers may specifically be evaluated in a chemometric approach, such as by using multivariate data analysis together with biomarker information from reference methods, e.g., the analysis of blood samples. Alternatively or additionally, classification or quantification algorithms can be applied, e.g., principle component analysis, least square regression, multiple linear regression.

### List of reference numbers

- 110: spectrometer
- 112: illumination source
- 114: sample
- 116: illumination light beam
- 118: light source
- 120: direction of propagation
- 122: sample interface
- 124: detector
- 126: direction of collection
- 128: dispersive element
- 130: baseline
- 132: optical sending fiber
- 134: optical receiving fiber
- 136: evaluation device
- 138: fraction of output power
- 140: angle of inclination
- 142: region-specific sampling
- 144: illuminating a sample of a subject
- 146: detecting light
- 148: evaluating the detected light

## Claims

1. A spectrometer (110) comprising
- at least one illumination source (112) configured for illuminating a sample (114) of a subject with at least one illumination light (116) beam having at least one wavelength in an optical spectral range, wherein the illumination source (112) is configured for transmitting the illumination light beam (116) to the sample (114) along a direction of propagation (120);
- at least one detector (124) sensitive to light in the optical spectral range, wherein the detector (124) is configured for detecting light impinging on the detector (124) under at least one direction of collection (126);
wherein the illumination source (112) and the detector (124) are separated by a baseline (130), wherein the direction of collection (126) and the direction of propagation (120) of the illumination light beam (116) form an angle of inclination α with α ≠ 0.

2. The spectrometer (110) according to the preceding claim, wherein the angle of inclination α is in the range of -85° ≤ α < 0°, preferably -85° ≤ α ≤ -50°.

3. The spectrometer (110) according to any one of the preceding claims, wherein the baseline (130) is from 1 to 5 mm, preferably the baseline (130) is ≥ 3 mm.

4. The spectrometer (110) according to any one of the preceding claims, wherein an aperture of the illumination source (112) and an aperture of the detector (124) are tilted with respect to each other.

5. The spectrometer (110) according to any one of the preceding claims, wherein the illumination source (112) and/or the detector (124) comprises at least one lens.

6. The spectrometer (110) according to any one of the preceding claims, wherein the illumination source (112) comprises at least one light source (118) configured for generating the illumination light beam (116), wherein the illumination source (112) comprises at least one optical sending fiber (132) configured for transmitting the illumination light beam (116) from the light source (118) to the sample (114).

7. The spectrometer (110) according to any one of the preceding claims, further comprising at least one dispersive element (128) arranged in front of the detector (124) in the direction of collection (126) of a light beam from the sample (114) to the detector (124).

8. The spectrometer (110) according to any one of the preceding claims, wherein the detector (124) comprises at least one optical receiving fiber (134) configured for receiving at least one incident light beam generated by the sample (114) upon interaction with the illumination light beam (116), and optionally transferring the light beam to a dispersive element (128).

9. The spectrometer (110) according to any one of the preceding claims, wherein the spectrometer (110) comprises at least one evaluation device (136) configured for evaluating the light detected by the detector (124), thereby determining at least one biomarker, wherein the biomarker is at least one biomarker selected from the group consisting of: body temperature, body hydration, hemoglobin oxygenation, concentration of glucose, concentration of lactate, concentration of ethanol, concentration of metabolic products of ethanol, e.g. aldehyde, collagen level, skin hydration, sebum (fat) concentration.

10. The spectrometer (110) according to any one of the preceding claims, wherein the detector (124) comprises at least one photosensitive element configured for generating at least one detector signal dependent on an illumination of its light-sensitive region with detection light and/or wherein the detector (124) comprises an array of photosensitive elements, wherein each of the photosensitive elements is configured for generating at least one detector signal dependent on an illumination of its light-sensitive region with detection light, wherein the spectrometer (110) is configured such that the photosensitive elements are sensitive to differing spectral ranges of the detection light.

11. A method for skin-layer specific spectroscopy using at least one spectrometer (110) according to any one of the preceding claims, comprising the following steps:
a. illuminating a sample (114) of a subject with at least one illumination light beam (116) having at least one wavelength in an optical spectral range by using the illumination source (112), wherein the illumination light beam (116) is transmitted to the sample (114) along a direction of propagation (120);
b. detecting light, generated by the sample (114) upon interaction with the illumination light beam (116), impinging on the detector (124) under at least one direction of collection (126);
c. evaluating the detected light to derive at least one item of spectral information on a subcutaneous region of the sample (114).

12. A computer program comprising instructions which, when the program is executed by the spectrometer (110) according to any one of the preceding claims referring to a spectrometer (110), cause the spectrometer (110) to perform the method according to any one of the preceding claims referring to a method.

13. A computer-readable storage medium comprising instructions which, when the instructions are executed by the spectrometer (110) according to any one of the preceding claims referring to a spectrometer (110) cause the spectrometer (110) to perform the method according to any one of the preceding claims referring to a method.

14. A non-transient computer-readable medium including instructions that, when executed by one or more processors, cause the one or more processors to perform the method according to any one of the preceding claims referring to a method.

15. A use of a spectrometer (110) according to any one of the preceding claims referring to a spectrometer (110) for skin-layer specific near infrared spectroscopy.
